(19) 

Europäisches
Patentamt
European
Patent Office
Office européen
des brevets

(11) **EP 3 683 277 A1**

(12) **EUROPEAN PATENT APPLICATION**
published in accordance with Art. 153(4) EPC

(43) Date of publication:
**22.07.2020 Bulletin 2020/30**

(21) Application number: **18857161.6**

(22) Date of filing: **14.09.2018**

(51) Int Cl.:
*C09B 61/00* (2006.01)  *A23L 5/43* (2016.01)
*A23L 5/46* (2016.01)  *A61K 8/96* (2006.01)

(86) International application number:
**PCT/JP2018/034149**

(87) International publication number:
**WO 2019/054472 (21.03.2019 Gazette 2019/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.09.2017 JP 2017177083**

(71) Applicant: **San-Ei Gen F.F.I., INC.
Toyonaka-shi, Osaka 561-8588 (JP)**

(72) Inventors:
• **YOKOTA,Aki**
**Toyonaka-shi**
**Osaka 561-8588 (JP)**
• **HAMASAKI,Koji**
**Toyonaka-shi**
**Osaka 561-8588 (JP)**
• **NAKASHIMA,Koichi**
**Toyonaka-shi**
**Osaka 561-8588 (JP)**

(74) Representative: **Cabinet Beau de Loménie
158, rue de l'Université
75340 Paris Cedex 07 (FR)**

(54) **METHOD FOR SUPPRESSING BUBBLING IN NATURALLY DERIVED WATER-SOLUBLE PIGMENT**

(57) The invention suppresses foaming that occurs when a naturally derived water-soluble pigment, such as spirulina blue, an anthocyanin-based pigment, a gardenia pigment, or a monascus pigment, is added to an aqueous solvent, such as water, and dissolved. A solid composition containing a naturally derived water-soluble pigment is subjected to a compression treatment.

[FIG. 1]

SAMPLE 1     SAMPLE 2

← LIQUID SURFACE

EP 3 683 277 A1

## Description

Technical Field

The present invention relates to a method for suppressing foaming of a naturally derived water-soluble pigment, and also to a method for producing a pigment preparation containing the pigment. More specifically, the invention relates to a method for suppressing foaming that occurs when a naturally derived water-soluble pigment in solid form, such as powder form or granular form, is dissolved in an aqueous solvent (e.g., water, etc.), and also to a method for producing a pigment preparation in which such foaming is suppressed.

Background Art

Conventionally, synthetic pigments and natural pigments (naturally derived pigments) have been widely used for coloring foods. Particularly in recent years, for their safe and healthy image, natural pigments are especially used. For example, spirulina blue is a naturally derived water-soluble bluish pigment that can color a target composition bright blue, and thus has been applied to foods and beverages.
As prior art related to a pigment preparation containing spirulina blue, PTL 1 proposes an aqueous pigment solution containing an algae-derived pigment, an alcohol, and water. PTL 2 proposes a powder pigment obtained by pulverizing a spirulina pigment to an average particle size of 3 to 8 μm. PTL 1 is a technology related to a pigment solution resistant to bacterial growth, while PTL 2 relates to a technology in which a powder pigment is added in powder form to color a food or beverage.

Prior Art Document

Patent Literature

PTL 1: JP-A-2004-231851
PTL 2: JP-A-2011-099105

Summary of Invention

Technical Problem

An object of the invention is to suppress foaming of a naturally derived water-soluble pigment, such as spirulina blue. As far as the applicant is aware, there has been no prior art example focusing on suppressing foaming of a naturally derived water-soluble pigment. Another object of the invention is to provide a pigment preparation, which contains a naturally derived water-soluble pigment and yet in which foaming is suppressed.

Solution to Problem

As a result of extensive research, the present inventors have found that when a solid composition containing a naturally derived water-soluble pigment is subjected to a compression treatment, foaming of the pigment can be suppressed. Based on such findings, the invention has been accomplished.
The invention encompasses the following embodiments.

Item 1. A method for suppressing foaming of a naturally derived water-soluble pigment, comprising a step of subjecting a solid composition containing the naturally derived water-soluble pigment to a compression treatment.
Item 2. The method for suppressing foaming according to item 1, wherein the solid composition is a powder composition or a granular composition.
Item 3. The method for suppressing foaming according to item 1 or 2, wherein the compression treatment is a roller compaction dry granulation treatment.
Item 4. The method for suppressing foaming according to any one of items 1 to 3, wherein the step of subjecting a solid composition to a compression treatment is performed in the presence of at least one member selected from the group consisting of disaccharides, stearates, and emulsifiers.
Item 5. The method for suppressing foaming according to any one of items 1 to 4, wherein the naturally derived water-soluble pigment is at least one member selected from the group consisting of spirulina blue, anthocyanin-based pigments, gardenia pigments, and monascus pigments.

Item 6. A method for producing a pigment preparation containing a naturally derived water-soluble pigment, the method comprising a step of subjecting a solid composition containing a naturally derived water-soluble pigment to a compression treatment.

Item 7. The method for producing a pigment preparation according to item 6, wherein the compression treatment is a roller compaction dry granulation treatment.

Item 8. The method for producing a pigment preparation according to item 6 or 7, wherein the pigment preparation has a color value within a range of 10 to 1,000.

Item 9. A pigment preparation comprising:

a naturally derived water-soluble pigment; and
at least one member selected from the group consisting of disaccharides, stearates, and emulsifiers, wherein the hardness is 1 to 30 N.

Item 10. A method for producing a colored composition,
the method for producing a colored composition comprising a step of coloring a composition using a pigment preparation containing a water-soluble pigment,
the pigment preparation being obtainable by a method comprising a step of subjecting a solid composition containing a naturally derived water-soluble pigment to a compression treatment.

Item 11. The method for producing a colored composition according to item 10, wherein the colored composition is an aqueous colored composition.

Item 12. The method for producing a colored composition according to claim 10 or 11, wherein the colored composition is at least one member selected from the group consisting of foods and beverages, perfumery and cosmetics, pharmaceuticals, quasi drugs, commodities, inks, and animal feeds.

Effects of Invention

According to the invention, foaming of a naturally derived water-soluble pigment can be suppressed.

Brief Description of Drawings

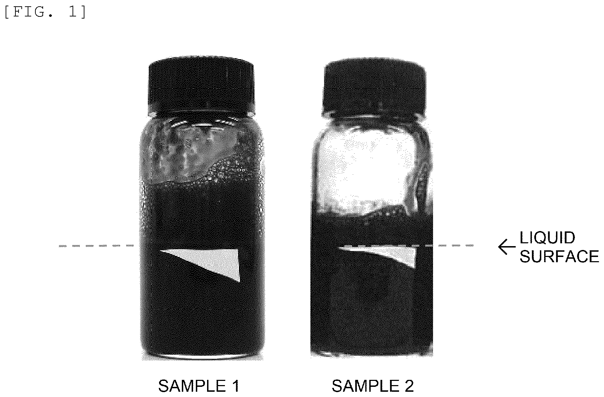

[Fig. 1] Fig. 1 is a photograph image showing the appearance of foaming after dissolving pigment preparations containing spirulina blue (Sample 1 and Sample 2) in water in Example 1 and Comparative Example 1.

Description of Embodiments

The invention provides:

a method for suppressing foaming, comprising a step of subjecting a solid composition containing a naturally derived water-soluble pigment to a compression treatment;
a method for producing a pigment preparation containing a naturally derived water-soluble pigment, comprising a step of subjecting a solid composition containing a naturally derived water-soluble pigment to a compression treatment; and
a method for producing a colored composition, comprising a step of coloring a composition using a pigment preparation containing a water-soluble pigment which is obtainable by a method comprising a step of subjecting a solid composition containing a naturally derived water-soluble pigment to a compression treatment.

According to the invention, when a naturally derived water-soluble pigment is added to an aqueous solvent such as water and dissolved, for example, foaming that occurs can be suppressed. Further, according to the invention, when a naturally derived water-soluble pigment is added to an aqueous solvent such as water and dissolved, excellent solubility can be exerted. Here, the exertion of excellent solubility refers to the maintenance or improvement of solubility, and means that, as compared to before compression treatment, the solubility is maintained, or even higher solubility can be exerted.

Hereinafter, the invention will be described in detail.

Terms

As used herein, the term "comprising" is used with the intention to encompass the terms "consisting essentially of" and "consisting of".

As used herein, as commonly understood by one of ordinary skill in the art in the field of pigments, "color value" is a numerical value that indicates the concentration of a pigment. A "color value" is expressed as a numerical value ($E^{10\%}_{1\ cm}$) obtained by calculating the absorbance of a pigment-containing composition, such as a pigment preparation, at the maximum absorption wavelength in the visible region in terms of the absorbance of a 10 w/v% solution.

In the invention, the color value ($E^{10\%}_{1\ cm}$) is a numerical value determined in accordance with Japan's Specifications and Standards for Food Additives, 8th Edition, "17. Color Value Measurement Method" in "B. GENERAL TEST" and also "C. MONOGRAPHS". For example, the value is determined by the following method.

(1) A test liquid is prepared such that the measured absorbance falls within a range of 0.3 to 0.7. For the preparation of a test liquid, a sample is precisely weighed, and a separately specified solvent is added thereto to accurately make 100 mL. Here, when precipitation or the like is observed, such a test liquid is centrifuged, and the resulting supernatant is used as a test liquid. In addition, in the case where a sample having a color value of 100 or more is used, the test liquid is diluted with a solvent as necessary such that the measured absorbance falls within a range of 0.3 to 0.7. Then, the dilution is used as a test liquid and subjected to the measurement.

(2) Using the solvent used to prepare the test liquid as a control, at the separately specified wavelength at a liquid layer length of 1 cm, the absorbance A at the maximum absorption wavelength is measured, and the color value ($E^{10\%}_{1\ cm}$) to be measured is determined by the following formula.

$$\text{Color value } (E^{10\%}_{1\ cm}) = A \times 10/\text{the amount of sample contained in 100 mL of the test liquid subjected to the measurement (g)}$$

The "separately specified solvent" and "separately specified wavelength" prescribed in the "17. Color Value Measurement Method" is prescribed in Japan's Specifications and Standards for Food Additives, 8th Edition, "C. MONOGRAPHS", which may be referenced as necessary. In addition, in the case where a test liquid preparation method is prescribed in Japan's Specifications and Standards for Food Additives, 8th Edition, "C. MONOGRAPHS", preparation can be performed in accordance with such prescription.

For reference, Table 1 shows the solvent used for each naturally derived water-soluble pigment and also the measurement wavelengths. A color value is a value widely used in the art and can be calculated using common knowledge in the art.

[Table 1]

| Pigment | Solvent | Wavelength |
|---|---|---|
| Spirulina blue | Citric acid buffer (pH 6.0) | 610 to 630 nm |
| Anthocyanin-based pigment | Citric acid buffer (pH 3.0) | 500 to 540 nm |
| Gardenia red | Citric acid buffer (pH 4.0) | 520 to 545 nm |
| Gardenia blue | Citric acid buffer (pH 7.0) | 570 to 610 nm |
| Monascus pigment | Water/ethanol mixture (1:1) | 450 to 520 nm |

In the invention, calculation in terms of color value means to calculate a pigment in terms of numerical value per color value.

Therefore, 1 part by mass in terms of a color value of 100 has the same meaning as 0.5 parts by mass in terms of a color value of 200 and 2 parts by mass in terms of a color value of 50.

[(I) Method for Suppressing Foaming of Naturally Derived Water-Soluble Pigment]

The method for suppressing foaming of a naturally derived water-soluble pigment of the invention (hereinafter sometimes referred to as the foaming suppressing method of the invention) is a method for suppressing foaming, comprising a step of subjecting a solid composition containing a naturally derived water-soluble pigment to a compression treatment.

In the invention, "suppressing foaming of a naturally derived water-soluble pigment" means to suppress foaming that occurs when a naturally derived water-soluble pigment in solid form (e.g., powder form, granular form, etc.) is dissolved in an aqueous solvent. In the invention, "suppress" means that as compared to before the compression treatment of the invention, foaming of a naturally derived water-soluble pigment after the treatment is reduced. Here, "aqueous solvent" encompasses water, lower alcohols, polyhydric alcohols, and mixtures of two or more kinds of them, and other components may further be contained. Preferable example of the aqueous medium may preferably be water, a polyhydric alcohol, a mixture of water and a polyhydric alcohol further containing other components, or the like. It is also preferable to use hydrous alcohol (for example, mixture of water and ethanol).

(Naturally Derived Water-Soluble Pigment)

The naturally derived water-soluble pigment, to which the foaming suppressing method of the invention is applied, is not particularly limited.

Naturally derived water-soluble pigments in the invention are water-soluble pigments derived from natural products, particularly those obtained by heat-treating a saccharide and those derived from algae or plants, and may be hereinafter sometimes simply referred to as water-soluble pigments. "Derived" means that the pigment is obtained by solvent extraction from algae or plants, for example. Examples of such water-soluble pigments include, but are not limited to:

algae-derived pigments such as spirulina blue;
anthocyanin-based pigments such as purple sweet potato pigments, red cabbage pigments, red radish pigments, red rice pigments, purple corn pigments, purple yam pigments, berry pigments, perilla pigments, cherry pigments, hibiscus pigments, grape juice pigments, grape skin pigments, black currant pigments, plum pigments, and red currant pigments;
gardenia pigments such as gardenia yellow, gardenia red, and gardenia blue;
water-soluble annatto pigments;
monascus pigments such as monascus red and monascus yellow;
carthamus pigments such as carthamus yellow and carthamus red;
red beet pigments;
flavonoid-based (polymer) pigments such as cacao pigments, Japanese persimmon pigments, and tamarind pigments;
quinone-based pigments such as cochineal extracts and lac pigments; and
kaoliang pigments and the like.

Among them, at least one pigment selected from the group consisting of spirulina blue, anthocyanin-based pigments, gardenia-based pigments, and monascus pigments is prone to foaming. In particular, spirulina blue is notably prone to foaming, and thus is a typical example of the naturally derived water-soluble pigment in the invention.

Spirulina blue is a pigment derived from the genus Spirulina and is a pigment composition containing phycocyanin as a main component. Spirulina blue can be acquired by, for example, extracting whole spirulina algae (Spirulina platensis Geitler) with water at room temperature, or it is also possible to use a commercially available product. The maximum absorption wavelength of spirulina blue is usually within a range of 610 to 630 nm. Such spirulina blue is preferable as a naturally derived water-soluble blue pigment and advantageous in that the target composition can be colored bright blue, but meanwhile has a problem of being notably prone to foaming. However, according to the invention, even when the targeting spirulina blue as a naturally derived water-soluble pigment, foaming can be significantly suppressed.

(Compression Treatment)

In the foaming suppressing method of the invention, a solid composition containing a naturally derived water-soluble pigment is subjected to a compression treatment. In one preferred embodiment of the invention, in terms of compression treatment efficiency, quality, and the like, the moisture content in the solid composition when subjected to the compression treatment is preferably 10 mass% or less, more preferably 8 mass% or less, still more preferably 7 mass% or less, and yet more preferably 6 mass% or less.

The lower limit of the moisture content in the solid composition is not particularly limited, but may be, for example, 0 mass%, 0.1 mass%, 0.2 mass%, 0.3 mass%, 0.4 mass%, or 0.5 mass%.

The moisture content in the solid composition can be determined by a (normal-pressure) heat drying method, for example. The moisture content can be adjusted according to the conventional method.

The solid composition containing a naturally derived water-soluble pigment can be obtained by subjecting a water-soluble pigment extraction liquid or production liquid extracted or produced from a natural product to a drying treatment. For example, in the case where a solid composition containing spirulina blue as a naturally derived water-soluble pigment is to be obtained, such a composition can be obtained by subjecting a spirulina blue extraction liquid extracted from spirulina algae to a drying treatment.

As the drying treatment, specifically, for example, a drying treatment by a spray-drying method, a vacuum-drying method (including a freeze-drying method), or the like can be exemplified. In addition, in the invention, as the solid composition, it is also possible to use a commercially available solid pigment (including a pigment preparation).

In the foaming suppressing method of the invention, the shape of the solid composition to be subjected to a compression treatment is not particularly limited, and may be powder form, granular form, or the like.

In the foaming suppressing method of the invention, the content of the naturally derived water-soluble pigment in the solid composition to be subjected to a compression treatment is not particularly limited, and can be suitably adjusted according to the kind of water-soluble pigment. According to one preferred embodiment of the invention, it is desirable that the solid composition contains a water-soluble pigment in such an amount that the color value of the solid composition is preferably 10 to 1,000, more preferably 20 to 800, and still more preferably 50 to 500.

The solid composition containing a naturally derived water-soluble pigment used in the invention can be a solid, naturally derived water-soluble pigment itself. In addition, as necessary, the solid composition can also contain fillers (or diluents), lubricants, emulsifiers, other additives, and the like in addition to the pigment.

Specific examples of the fillers (or diluents) described above include saccharides (e.g., monosaccharides such as glucose, galactose, and fructose; disaccharides such as trehalose, sucrose, lactose, and maltose; oligosaccharide, starch syrup, dextrin, cyclodextrin, etc.) and sugar alcohols (e.g., sorbitol, mannitol, xylitol, erythritol, maltitol, etc.).

The total content of fillers (or diluents) in the solid composition in the case of using fillers (or diluents) is not particularly limited, but the content is preferably 10 to 90 mass%, more preferably 20 to 80 mass%, still more preferably 30 to 70 mass%, and particularly preferably 40 to 60 mass%.

Specific examples of the lubricants described above include calcium stearate, magnesium stearate, talc, hardened oils (e.g., hardened rapeseed oil, etc.), beeswax, sucrose fatty acid esters, polyglycerin fatty acid esters, sodium stearyl fumarate, calcium silicate, magnesium silicate, silicon dioxide, silica hydrogel, leucine, isoleucine, and polyethylene glycol.

The total content of lubricants in the solid composition in the case of using lubricants is not particularly limited, but the content is preferably 0.02 to 6 mass%, more preferably 0.05 to 5 mass%, still more preferably 0.08 to 4 mass%, and yet more preferably 0.1 to 3 mass%.

Specific examples of the emulsifiers described above include glycerin fatty acid esters (e.g., glycerin fatty acid monoglycerides, organic acid monoglycerides, polyglycerin fatty acid esters, polyglycerin condensed ricinoleic acid esters, etc.), propylene glycol fatty acid esters, sucrose fatty acid esters, sorbitan monostearate, sorbitan tristearate, polysorbate, fatty acid soaps (Na salt, K salt, Ca salt), sodium stearoyl lactylate, calcium stearoyl lactylate, saponin, and lecithins (e.g., soybean lecithin, yolk lecithin, fractionated lecithin, enzymatically decomposed lecithin, enzymatically modified lecithin, etc.) .

The emulsifier content in the solid composition in the case of using emulsifiers is not particularly limited, but the content is preferably 0.05 to 12 mass%, more preferably 0.08 to 10 mass%, still more preferably 0.1 to 10 mass%, yet more preferably 0.2 to 8 mass%, and particularly preferably 0.2 to 5 mass%.

Examples of the other additives described above include antioxidants, chelating agents, flavoring agents, spice extracts, and preservatives.

In the foaming suppressing method of the invention, the compression treatment is not particularly limited as long as it is a treatment according to which the solid composition containing a naturally derived water-soluble pigment is compressed. In the invention, "compress" means that the solid composition after treatment has a higher bulk specific gravity than before treatment. Examples of compression treatments include a compression treatment using a tableting press or the like, a compression treatment using a roller compactor or the like, and an extrusion granulation method. The compression treatment is preferably a compression treatment using a tableting press or the like or a compression treatment using a roller compactor or the like.

Roller compaction dry granulation is a granulation method also called a roller compactor method. Two vertically or horizontally arranged rolls are rotated in opposite directions from each other, then a granulation raw material (corresponding to the solid composition in the invention) is fed between the rolls, and a high pressure is applied from the rolls to compress the material into sheet form. The formed sheet is finely crushed in a crusher and sieved as necessary, thereby giving grains. Such roller compaction dry granulation is a dry granulation method using no granulation liquid such as water.

In the foaming suppressing method of the invention, the pressure condition in the compression treatment is not particularly limited. In one preferred embodiment of the invention, the preferred pressure condition is 1 to 20 MPa, preferably 2 to 15 MPa, and more preferably 3 to 10 MPa. Such a pressure may be, for example, the set pressure of the tableting press or roll used for the compression treatment.

In the invention, it is preferable that the compression treatment is performed in the presence of at least one member selected from the group consisting of disaccharides, stearates, and emulsifiers. Among them, it is particularly preferable that the compression treatment is performed in the presence of at least one member selected from the group consisting of stearates, lactose, maltose, and sucrose esters. Here, as stearates, calcium stearate or magnesium stearate is preferable, and magnesium stearate is particularly preferable.

The at least one member selected from the group consisting of disaccharides, stearates, and emulsifiers may be previously contained in the pigment-containing solid composition before the compression treatment to serve also as a filler (or diluent), a lubricant, and an emulsifier, or may also be added at the time of the compression treatment.

The total amount of at least one member selected from the group consisting of disaccharides, stearates, and emulsifiers contained at the time of the compression treatment is not particularly limited, but the amount is preferably 0.02 to 6 mass%, more preferably 0.05 to 5 mass%, still more preferably 0.08 to 4 mass%, yet more preferably 0.1 to 3 mass%, and particularly preferably 0.5 to 1 mass%.

The total amount of at least one member selected from the group consisting of stearates, lactose, maltose, and sucrose esters contained at the time of the compression treatment is not particularly limited, but the amount is preferably 0.02 to 6 mass%, more preferably 0.05 to 5 mass%, still more preferably 0.08 to 4 mass%, yet more preferably 0.1 to 3 mass%, and particularly preferably 0.5 to 1 mass%.

According to the foaming suppressing method of the invention, when a naturally derived water-soluble pigment in solid form is dissolved in an aqueous solvent, foaming that occurs can be notably suppressed.

A naturally derived water-soluble pigment in solid form (e.g., powder form, granular form, etc.) is advantageous over a liquid water-soluble pigment in that the storage stability is excellent (e.g., bacteria are less likely to grow, the color value is less likely to decrease, etc.), the distribution cost is low, and the like. However, there is a problem in that when a solid water-soluble pigment is added to an aqueous solvent and dissolved, foam is formed on top of the liquid surface of the aqueous solvent, and the foam once formed does not disappear easily.

However, according to the foaming suppressing method of the invention, when a naturally derived water-soluble pigment in solid form is dissolved in an aqueous solvent, foaming that occurs can be suppressed.

In particular, in the case where the naturally derived water-soluble pigment is dissolved at a high concentration in an aqueous solvent, for example, in the case where the pigment is dissolved such that the pigment concentration relative to water in the aqueous solvent is 3 mass% or more, 5 mass% or more, 7 mass% or more, or 10 mass% or more in terms of a color value of 100, foaming is even more likely to occur.

Conventionally, silicone is known as a kind of defoaming agent. However, in the case where a naturally derived water-soluble pigment is dissolved at a high concentration in an aqueous solvent as described above, foaming cannot be sufficiently suppressed.

However, according to the foaming suppressing method of the invention, foaming can be suppressed even in the case where a naturally derived water-soluble pigment is dissolved at a high concentration in an aqueous solvent as described above. This effect is a remarkable effect that cannot be anticipated from the prior art.

The upper limit of the pigment concentration in the case of dissolving a naturally derived water-soluble pigment at a high concentration in an aqueous solvent is not particularly limited, and may be, for example, as the pigment concentration relative to water in the aqueous solvent, 70 mass% or less, preferably 50 mass% or less, in terms of a color value of 200.

As used herein, calculation "in terms of color value" means to calculate a pigment in terms of numerical value per color value.

Usually, spirulina blue is not a single compound (a pigment composition containing phycocyanin), and thus the concentration of spirulina blue is expressed as a color value. Similarly, an anthocyanin-based pigment, a gardenia pigment, a monascus pigment, and the like are not single compounds either, and thus their concentrations are usually expressed as color values.

For example, "10 mass% in terms of a color value of 100" means such an amount that in the case where a pigment is adjusted to make a color value of 100, the pigment content in the composition is 10 mass%. Therefore, "10 mass% in terms of a color value of 100" has the same meaning as, for example, containing 5 mass% of a pigment having a color value of 200 (10 mass% $\times$ color value ratio 0.5 (100/200)) or containing 20 mass% of a pigment having a color value of 50 (10 mass% $\times$ color value ratio 2 (100/50)).

[(II) Method for Producing Pigment Preparation Containing Naturally Derived Water-Soluble Pigment]

According to the foaming suppressing method of the invention described above, a composition containing a naturally derived water-soluble pigment, in which foaming upon dissolution in an aqueous solvent is suppressed, can be produced.

The composition containing a naturally derived water-soluble pigment obtained through a compression treatment can be formed into a pigment preparation for coloring various compositions and is useful for various applications.

That is, one embodiment of the invention is a method for producing a pigment preparation containing a naturally derived water-soluble pigment, particularly a method for producing a pigment preparation containing a naturally derived water-soluble pigment, in which foaming of the pigment is suppressed.

The method for producing a pigment preparation containing a naturally derived water-soluble pigment of the invention can be implemented in accordance with the method described above in "(I) Method for Suppressing Foaming of Naturally Derived Water-Soluble Pigment".

In accordance with the method described above in "(I) Method for Suppressing Foaming of Naturally Derived Water-Soluble Pigment", a composition prepared by subjecting a solid composition containing a naturally derived water-soluble pigment to a compression treatment (hereinafter sometimes referred to be "compression-treated composition") is obtained.

In the method for producing a pigment preparation of the invention, the compression-treated composition containing a naturally derived water-soluble pigment obtained through a compression treatment may be directly used as a pigment preparation, or it is also possible to optionally add fillers (or diluents), lubricants, emulsifiers, other additives, and the like. As fillers (or diluents), lubricants, emulsifiers, other additives, and the like, those described above in "(I) Method for Suppressing Foaming of Naturally Derived Water-Soluble Pigment" can be used.

[(III) Pigment Preparation Containing Naturally Derived Water-Soluble Pigment]

According to the method for producing a pigment preparation of the invention, a solid pigment preparation, which contains a naturally derived water-soluble pigment and in which foaming that occurs when the naturally derived water-soluble pigment is dissolved in an aqueous solvent is suppressed, is obtained.

The content of the naturally derived water-soluble pigment in the pigment preparation of the invention is not particularly limited, but it is desirable to contain a water-soluble pigment in such an amount that the color value ($E^{10\%}_{1\,cm}$) of the pigment preparation is preferably 10 to 1,000, more preferably 20 to 800, and still more preferably 50 to 500.

The shape or the like of the pigment preparation of the invention is not particularly limited, and it is possible that a composition prepared by subjecting a solid composition containing a naturally derived water-soluble pigment to a compression treatment is used in the form of tablets or sheets, for example. However, in terms of foaming suppression and solubility in an aqueous solvent, it is desirable that the preparation is in granular form. Such granules can be obtained, for example, by suitably grinding a composition prepared by subjecting a solid composition to a compression treatment.

The pigment preparation obtained from a composition prepared by subjecting a solid composition containing a naturally derived water-soluble pigment to a compression treatment has a hardness of 1 to 30 N, preferably 5 to 25 N. The hardness measurement method is as described in detail in the Examples.

The particle size of the pigment preparation of the invention can be suitably adjusted according to the intended use and purpose of the pigment preparation, and is not particularly limited.

For example, as one preferred embodiment of the invention, the following conditions can be mentioned; the proportion of particles passing through a sieve having a mesh size of 1,700 μm (10-mesh) is 50 mass% or more, preferably 70 mass% or more, and more preferably 80 mass% or more.

The upper limit of the proportion of particles passing through a 10-mesh sieve is not particularly limited, and may be 100 mass%, for example.

In addition, as one preferred embodiment of the invention, the following conditions can be mentioned; the proportion of particles passing through a sieve having a mesh size of 75 μm (200-mesh) is 50 mass% or less, preferably 40 mass% or less, and still more preferably 30 mass% or less.

The lower limit of the proportion of particles passing through a 200-mesh sieve is not particularly limited, and may be 0 mass%, for example.

A pigment preparation that satisfies the above conditions can be obtained by suitably grinding a composition prepared by subjecting a solid composition containing a naturally derived water-soluble pigment to a compression treatment.

The moisture content in the pigment preparation according to the invention is not particularly limited. However, in terms of storage stability and the like, the moisture content is preferably 10 mass% or less, more preferably 8 mass% or less, still more preferably 7 mass% or less, and still more preferably 6 mass% or less. The lower limit of the moisture content is not particularly limited, and it is possible that no moisture is contained, or the moisture content is 1 mass% or more.

In the invention, it is preferable that the pigment preparation contains at least one member selected from the group consisting of disaccharides, stearates, and emulsifiers. Among them, it is particularly preferable to contain at least one member selected from the group consisting of stearates, lactose, maltose, and sucrose esters. Here, as stearates, calcium stearate and magnesium stearate are preferable, and magnesium stearate is particularly preferable.

The at least one member selected from the group consisting of disaccharides, stearates, and emulsifiers may serve as a filler (or diluent), a lubricant, and an emulsifier.

The amount of at least one member selected from the group consisting of disaccharides, stearates, and emulsifiers contained in the pigment preparation is not particularly limited, but the amount is preferably 0.02 to 6 mass%, more preferably 0.05 to 5 mass%, still more preferably 0.08 to 4 mass%, yet more preferably 0.1 to 3 mass%, and particularly preferably 0.5 to 1 mass%.

[(IV) Method for Producing Colored Composition]

The invention also relates to a method for producing a colored composition, comprising a step of coloring a composition using the pigment preparation described above (hereinafter sometimes referred to as the method for producing a colored composition of the invention).

In the invention, the composition to be colored is not particularly limited, and includes various compositions such as foods and beverages, perfumery and cosmetics, pharmaceuticals, quasi drugs, commodities, inks, and animal feeds. According to one preferred embodiment of the invention, the composition is a food or beverage. In the invention, the composition is also particularly preferably, but is not limited to, an aqueous colored composition such as a sugarcoating syrup, a beverage, a jelly, or a frozen dessert.

Examples of the foods and beverages described above include:

beverages such as beverages and alcoholic beverages;
confectionaries such as frozen desserts (e.g., ice pops, ice creams, etc.), sugar confectioneries (e.g., candies, gummies, marshmallows, chewing gums, chocolates, etc.), patisseries (e.g., cakes, cookies, macaroons, jellies, puddings, Bavarian creams, etc.), snacks, Japanese sweets (e.g., rice dumplings, rice crackers, doughnuts, sponge cakes, etc.);
processed agricultural products such as dried vegetables and pickles;
processed marine products;
processed grain products such as noodles, cooked rice, and bread;
seasonings;
syrups, jams, and the like; and
processed livestock meat products.

Examples of the perfumery and cosmetics described above include cosmetic products such as make-up products (e.g., eye shadow, mascara, lip stick, etc.), skin care products, hair care products (e.g., hair color, etc.), and oral care products (e.g., tooth paste, mouthwash, etc.); perfumes, lip balms, and bath fragrances.

Examples of the pharmaceuticals described above include tablets (e.g., sugar-coated tablets), granules, liquids, capsules, troches, and gargles.

Examples of the quasi drugs described above include nutritious supplement, various supplements, mouth deodorants, mouth refrigerants, hair growth stimulants, and hair restorers.

Examples of the commodities described above include pesticides, insecticides, dehumidifying agents, deodorants, and detergents.

Examples of the inks described above include dye inks and printing inks.

Examples of the animal feeds described above include pet foods such as cat foods, dog foods, and fish foods.

The step of coloring the composition can be performed in the conventional method for each composition, except for that the pigment preparation according to the invention is blended. The blending proportion of the pigment preparation according to the invention in each composition can be suitably adjusted according to the kind of composition or the purpose. For example, in one preferred embodiment of the invention, the pigment preparation can be blended such that the content of the pigment preparation in the composition is 0.001 to 5 mass%, preferably 0.005 to 3 mass%, more preferably 0.01 to 1.5 mass%, and still more preferably 0.03 to 1 mass% in terms of a color value of 100.

When each composition is colored using a naturally derived water-soluble pigment, in the case where the amount of water that dissolves the pigment is small, that is, in the case where the amount of naturally derived water-soluble pigment is large relative to water, foaming of the pigment tends to be even more notable.

For example, in the case where the naturally derived water-soluble pigment is dissolved such that the pigment concentration relative to water is 3 mass% or more, further 5 mass% or more, particularly 7 mass% or more, or especially 10 mass% or more in terms of a color value of 100, foaming is even more likely to occur. However, according to the method for producing a colored composition of the invention, in the step of coloring a composition using the pigment preparation, even in the case where the blending amount of naturally derived water-soluble pigment relative to water is 10 mass% or more in terms of a color value of 100, foaming of the pigment is suppressed, and, as a result, a colored composition can be efficiently produced.

The upper limit of the concentration of the naturally derived water-soluble pigment is not particularly limited, and may be, for example, as the concentration relative to water in the aqueous solvent, 70 mass% or less, preferably 50 mass% or less, in terms of a color value of 200.

The invention encompasses the following embodiments.

[1] A method for suppressing foaming of a naturally derived water-soluble pigment, comprising a step of subjecting a solid composition containing the naturally derived water-soluble pigment to a compression treatment.

[2] The method for suppressing foaming according to [1], wherein the solid composition is a powder composition or a granular composition.

[3] The method for suppressing foaming according to [1] or [2], wherein the compression treatment is a roller compaction dry granulation treatment.

[4] The method for suppressing foaming according to any one of [1] to [3], wherein the step of subjecting a solid composition to a compression treatment is performed in the presence of at least one member selected from the group consisting of disaccharides, stearates, and emulsifiers.

[5] The method for suppressing foaming according to [4], wherein the disaccharide is lactose or maltose.

[6] The method for suppressing foaming according to [4], wherein the emulsifier is a sucrose fatty acid ester.

[7] The method for suppressing foaming according to any one of [1] to [6], wherein the naturally derived water-soluble pigment is at least one member selected from the group consisting of spirulina blue, anthocyanin-based pigments, gardenia pigments, and monascus pigments.

[8] A method for producing a pigment preparation containing a naturally derived water-soluble pigment,

the method comprising a step of subjecting a solid composition containing a naturally derived water-soluble pigment to a compression treatment.

[9] The method for producing a pigment preparation according to [8], wherein the compression treatment is a roller compaction dry granulation treatment.

[10] The method for producing a pigment preparation according to [8] or [9], wherein the pigment preparation has a color value within a range of 10 to 1,000.

[11] The method for producing a pigment preparation according to any one of [8] to [10], wherein the step of subjecting a solid composition to a compression treatment is performed in the presence of at least one member selected from the group consisting of disaccharides, stearates, and emulsifiers.

[12] The method for producing a pigment preparation according to [11], wherein the disaccharide is lactose or maltose.

[13] The method for producing a pigment preparation according to [11], wherein the emulsifier is a sucrose fatty acid ester.

[14] The method for producing a pigment preparation according to any one of [8] to [13], wherein the naturally derived water-soluble pigment is at least one member selected from the group consisting of spirulina blue, anthocyanin-based pigments, gardenia pigments, and monascus pigments.

[15] A pigment preparation obtainable by a method comprising a step of subjecting a solid composition containing a naturally derived water-soluble pigment to a compression treatment.

[16] The pigment preparation according to [15], wherein the step of subjecting a solid composition to a compression treatment is performed in the presence of at least one member selected from the group consisting of disaccharides, stearates, and emulsifiers.

[17] The pigment preparation according to [16], wherein the disaccharide is lactose or maltose.

[18] The pigment preparation according to [16], wherein the emulsifier is a sucrose fatty acid ester.

[19] The pigment preparation according to any one of [15] to [18], wherein the naturally derived water-soluble pigment is at least one member selected from the group consisting of spirulina blue, anthocyanin-based pigments, gardenia pigments, and monascus pigments.

[20] A method for producing a colored composition,

the method comprising a step of coloring a composition using a pigment preparation containing a water-soluble pigment,

wherein the pigment preparation is obtainable by a method comprising a step of subjecting a solid composition containing a naturally derived water-soluble pigment to a compression treatment.

[21] The method for producing a colored composition according to [20], wherein the colored composition is an aqueous colored composition.

[22] The method for producing a colored composition according to [20] or [21], wherein the colored composition is at least one member selected from the group consisting of foods and beverages, perfumery and cosmetics, pharmaceuticals, quasi drugs, commodities, inks, and animal feeds.

[23] A pigment preparation comprising:

a naturally derived water-soluble pigment; and
at least one member selected from the group consisting of disaccharides, stearates, and emulsifiers, and having a hardness of 1 to 30 N.

Examples

Hereinafter, the invention will be described with reference to examples. However, the scope of the invention is not limited to these examples.

Unless otherwise noted, the experiment examples were all performed under room temperature conditions.

(Example 1) Production of Pigment Preparation Containing Spirulina Blue

A solid composition containing spirulina blue was subjected to a compression treatment using a tableting press, thereby producing a pigment preparation containing spirulina blue as a naturally derived water-soluble pigment.

(Material)

Pigment preparation containing spirulina blue (Sample 1)

"LINA BLUE G1" (powder), manufactured by DIC Lifetech Co., Ltd., color value: 185, moisture content: 6 mass% or less, breakdown: 40 mass% of spirulina blue, 55 mass% of filler (trehalose), and 5 mass% of trisodium citrate.

(Compression Conditions)

Tableting press: "HANDTAB-200", manufactured by Ichihashi Seiki Co., Ltd.
Pressure: 4 MPa (pestle diameter: 18 mm, Sample 1: 0.3 g, force: 2 tf (deadweight ton))

(Production Method)

The Sample 1 was subjected to a compression treatment using a tableting press to produce a solid compression-treated composition. The compression-treated composition was ground in a mortar, and passed through a 10-mesh sieve and then through a 30-mesh sieve. Granules remaining on the 30-mesh sieve were recovered to give a granular compression-treated composition (color value: 185). The average particle size (median size) of the granular compression-treated composition was within a range of 500 $\mu$m or more and 1,700 $\mu$m or less. The obtained granular compression-treated composition was used as Sample 2 (pigment preparation containing spirulina blue) and subjected to the following foaming test.

<Spirulina Blue Foaming Test>

Foaming that occurs when spirulina blue is added to water and dissolved was examined.

Two 30-mL volume screw tubes were prepared. 5 g of the solid compositions containing spirulina blue (Sample 1 and Sample 2) were added to the screw tubes, respectively, and then water (ion-exchange water) was poured. The amount of water poured was such that the spirulina blue concentration relative to water was 55.5 mass% in terms of a color value of 100 (30 mass% of the compression-treated composition having a color value of 185).

The mixture was hand stirred using a medicine spoon under conditions of 120 to 150 rpm until complete dissolution, and foaming of spirulina blue was visually checked. Here, the evaluation using Sample 1 is Comparative Example 1, while the evaluation using Sample 2 is Example 1.

(Evaluation of Foaming)

With respect to each screw tube, the height from the liquid surface to the top of foam was measured. In addition, in order to compare the degree of foaming, the foam percentage was calculated as follows. A lower percentage indicates a lower degree of foaming.

Table 2 shows the resulting measured values, and Fig. 1 shows the appearance of the screw tubes after dissolving spirulina blue.

$$(\text{Foam percentage}) = \text{foam part (height from the liquid surface to the top of foam)/liquid part (height from the screw tube bottom surface to the liquid surface)} \times 100\ (\%)$$

[Table 2]

| Used Sample | | Height from Liquid Surface to Foam Top | Foam Part/ Liquid Part |
|---|---|---|---|
| Comparative Example 1 | Non-compressed composition | 2.8 cm | 104% |
| Example 1 | Compression-treated composition | 0.7 cm | 26% |

As can be appreciated from Table 2 and Fig. 1, in the pigment preparation containing spirulina blue of Comparative Example 1, the amount of foam generated was almost the same as the liquid amount. In contrast, in the pigment preparation containing spirulina blue of Example 1 according to the invention (compressed composition), the amount of foam generated was about 1/4 or less the liquid amount.
The above results show that foaming of spirulina blue, which is a naturally derived water-soluble pigment, can be notably suppressed by going through a compression treatment step.

(Example 2) Production of Pigment Preparation Containing Spirulina Blue

A solid composition containing spirulina blue was subjected to a compression treatment using a roller compactor, thereby producing a pigment preparation containing spirulina blue as a naturally derived water-soluble pigment.

(Material)

Pigment preparation containing spirulina blue (Sample 1)

(Compression Conditions)

Roller compactor: "ROLLER COMPACTOR TF-MINI", manufactured by Freund Corporation
Pressure: 5 MPa

The Sample 1 was subjected to a compression treatment using a roller compactor to produce a solid (granular) compression-treated composition (color value: 185). The compression-treated composition was passed through a 10-mesh sieve and then through a 200-mesh sieve. As a result, the proportion of particles passing through the 10-mesh sieve was 100 mass%, and the proportion of particles passing through the 200-mesh sieve was 13 mass%.
The obtained granular compression-treated composition was used as Sample 3 (Example 2 : pigment preparation containing spirulina blue) and subjected to a foaming test.

<Spirulina Blue Foaming Test>

In accordance with the test of Example 1, Sample 1 and Sample 3 were dissolved in water, and foaming that occured when spirulina blue was added to water and dissolved was examined. In the test, the mixture was hand stirred using a spatula under conditions of 40 to 50 rpm until complete dissolution, and foaming of spirulina blue was visually checked. Here, the evaluation using Sample 1 is Comparative Example 2, while the evaluation using Sample 3 is Example 2. Table 3 shows the resulting measured values.

[Table 3]

| Used Sample | | Height from Liquid Surface to Foam Top | Foam Part/ Liquid Part |
|---|---|---|---|
| Comparative Example 2 | Non-compressed composition | 1.5 cm | 62.5% |
| Example 2 | Compression-treated composition | 0.5 cm | 20% |

As can be appreciated from Table 3, in the pigment preparation containing spirulina blue of Sample 1, the amount of foam generated was at least half the liquid amount. In contrast, in the pigment preparation containing spirulina blue of Sample 3 according to the invention (compressed composition), the amount of foam generated was 1/3 or less that in Comparative Example 2.

The above results show that foaming of spirulina blue, which is a naturally derived water-soluble pigment, can be notably suppressed by going through a compression treatment step.

(Example 3) Production of Pigment Preparation Containing Spirulina Blue

A solid composition containing spirulina blue was subjected to a compression treatment using a roller compactor, thereby producing a pigment preparation containing spirulina blue as a naturally derived water-soluble pigment.

(Material)

Pigment preparation containing spirulina blue (Sample 1)

(Compression Conditions)

Roller compactor: "ROLLER COMPACTOR TF-MINI", manufactured by Freund Corporation
Pressure: 5 MPa

600 g of the Sample 1 and 6 g of magnesium stearate were mixed in powder, and the mixture was subjected to a compression treatment using a roller compactor to produce a solid compression-treated composition (color value: 185). The compression-treated composition was passed through a 10-mesh sieve and then through a 200-mesh sieve. As a result, the proportion of particles passing through the 10-mesh sieve was 100 mass%, and the proportion of particles passing through the 200-mesh sieve was 13 mass%.

The obtained granular compression-treated composition was used as the pigment preparation of Example 3 and subjected to hardness measurement.

(Examples 4 to 11) Production of Pigment Preparation Containing Spirulina Blue

A solid composition containing spirulina blue was subjected to a compression treatment using a roller compactor, thereby producing a pigment preparation containing spirulina blue as a naturally derived water-soluble pigment.

(Material)

Pigment preparation containing spirulina blue (Sample 1)

(Compression Conditions)

Roller compactor: "ROLLER COMPACTOR TF-Labo", manufactured by Freund Corporation
Pressure: 5 MPa

The disaccharide, stearate, and emulsifier shown in Table 4 were added to the Sample 1, and subjected to a compression treatment using a roller compactor, thereby producing solid (granular) compression-treated compositions (color value: 185). The compression-treated compositions were each passed through a 10-mesh sieve and then through a 200-mesh sieve. As a result, the proportion of particles passing through the 10-mesh sieve was 100 mass%, and the proportion of particles passing through the 200-mesh sieve was 13 mass%. Incidentally, in the table, the numerical values are mass%, and the number of grams at the time of preparation is directly reflected.

The obtained granular compression-treated compositions were subjected to a foaming test and a solubility test. The sample of Example 4 was also subjected to hardness measurement.

[Table 4]

| (mass %) | | | | | | | | |
|---|---|---|---|---|---|---|---|---|
| | Example 4 | Example 5 | Example 6 | Example 7 | Example 8 | Example 9 | Example 10 | Example 11 |
| Spirulina blue pigment preparation (Sample 1) | 99.9 | 99 | 99.5 | 99.5 | 99 | 99 | 99.9 | 99 |
| Lactose | 0.1 | - | 0.5 | - | - | - | - | 0.5 |
| Mg stearate | - | 1 | - | - | - | - | - | - |
| Maltose | - | - | - | 0.5 | 1 | - | - | - |
| Sucrose fatty acid ester | - | - | - | - | - | 1 | 0.1 | 0.5 |
| Total | 100 | 100 | 100 | 100 | 100 | 100 | 100 | 100 |
| Lactose: TABLETTOSE 80 (available from Meggle Japan Co. Ltd.)<br>Maltose: SUNMALT-S (manufactured by Hayashibara Co., Ltd.): Example 7<br>SUNMALT-MIDORI (manufactured by Hayashibara Co., Ltd.): Example 8 Sucrose fatty acid ester: DK Ester F-160 (manufactured by DKS Co., Ltd.) | | | | | | | | |

< Hardness Measurement of Spirulina Blue Pigment Preparation>

The hardness of a spirulina blue pigment preparation was measured in accordance with JIS "Crushing Strength Test Method" of "Granules and Agglomerated-Test Methods for Strength" (JIS Z8841). A value (unit: N) measured using the following measurement device under the following measurement conditions was defined as hardness.

(Measurement Device)

Texture analyzer TA-XTplus (Stable Micro System)

(Measurement Conditions)

Target mode: Strain (distortion factor: 100%)
Speed: 0.15 mm/s
Plunger size: KOBE 1 cm$^2$ CYLINDER STAINLESS 1 cm$^2$ (cylindrical)

Specifically, the contact surface of the plunger to granules is a parallel plane, and the pressurization rate is about 0.15 to 0.3 mm/s.

Return to start (= one-time push test)
Target mode: Strain

Considering variation in granules, pressurization was controlled with Strain (distortion factor). The distortion factor was set at 100%, depending on the height of each granule, it was pushed in by the height.

Speed: 0.15 mm/s (for before, during, and after pressurization of granules)

The results of hardness measurement will be shown. In the measurement, five granules were taken, and their average was determined.

[Table 5]

|  | Example 2 | Example 3 | Example 4 |
|---|---|---|---|
| Force (N) | 24.998 | 18.223 | 5.286 |

<Foaming Test and Solubility Evaluation of Spirulina Blue Pigment Preparation>

In the same manner as in Example 2, the spirulina blue pigment preparations of Examples 4 to 11 were subjected to a foaming test and solubility evaluation.

[Table 6]

| | | Example 4 | Example 5 | Example 6 | Example 7 | Example 8 | Example 9 | Example 10 | Example 11 |
|---|---|---|---|---|---|---|---|---|---|
| Solubility (Dissolution time) | | 6 min 45 sec | 8 min | 8 min 45 sec | 7 min 52 sec | 7 min 8 sec | 5 min 40 sec | 7 min 15 sec | 6 min 50 sec |
| Foaming | Foam (cm) | 0.7 | 0.6 | 0.7 | 0.7 | 0.7 | 0.7 | 0.6 | 0.7 |
| | Liquid (cm) | 2.5 | 2.5 | 2.5 | 2.4 | 2.4 | 2.5 | 2.6 | 2.4 |
| | Foam percentage (%) | 28 | 24 | 28 | 29.2 | 29.2 | 28.0 | 23.1 | 29.2 |

In the composition containing spirulina blue before the compression treatment (Sample 1), the solubility (dissolution time) was 8 minutes and 30 seconds, and the foaming was 62.5%. Comparing this result with the numerical values shown in Table 4, it can be appreciated that the composition of the example has excellent properties both in solubility and foaming suppression.

## Claims

1. A method for suppressing foaming of a naturally derived water-soluble pigment, comprising a step of subjecting a solid composition containing the naturally derived water-soluble pigment to a compression treatment.

2. The method for suppressing foaming according to claim 1, wherein the solid composition is a powder composition or a granular composition.

3. The method for suppressing foaming according to claim 1 or 2, wherein the compression treatment is a roller compaction dry granulation treatment.

4. The method for suppressing foaming according to any one of claims 1 to 3, wherein the step of subjecting a solid composition to a compression treatment is performed in the presence of at least one member selected from the group consisting of disaccharides, stearates, and emulsifiers.

5. The method for suppressing foaming according to any one of claims 1 to 4, wherein the naturally derived water-soluble pigment is at least one member selected from the group consisting of spirulina blue, anthocyanin-based pigments, gardenia pigments, and monascus pigments.

6. A method for producing a pigment preparation containing a naturally derived water-soluble pigment, the method comprising a step of subjecting a solid composition containing a naturally derived water-soluble pigment to a compression treatment.

7. The method for producing a pigment preparation according to claim 6, wherein the compression treatment is a roller compaction dry granulation treatment.

8. The method for producing a pigment preparation according to claim 6 or 7, wherein the pigment preparation has a color value within a range of 10 to 1,000.

9. A pigment preparation comprising:

   a naturally derived water-soluble pigment; and
   at least one member selected from the group consisting of disaccharides, stearates, and emulsifiers, and
   wherein the hardness is 1 to 30 N.

10. A method for producing a colored composition, the method for producing a colored composition comprising a step of coloring a composition using a pigment preparation containing a water-soluble pigment, the pigment preparation being obtainable by a method comprising a step of subjecting a solid composition containing a naturally derived water-soluble pigment to a compression treatment.

11. The method for producing a colored composition according to claim 10, wherein the colored composition is an aqueous colored composition.

12. The method for producing a colored composition according to claim 10 or 11, wherein the colored composition is at least one member selected from the group consisting of foods and beverages, perfumery and cosmetics, pharmaceuticals, quasi drugs, commodities, inks, and animal feeds.

[FIG. 1]

SAMPLE 1        SAMPLE 2

← LIQUID
   SURFACE

## INTERNATIONAL SEARCH REPORT

| | International application No. |
|---|---|
| | PCT/JP2018/034149 |

**A. CLASSIFICATION OF SUBJECT MATTER**
Int.Cl. C09B61/00(2006.01)i, A23L5/43(2016.01)i, A23L5/46(2016.01)i,
A61K8/96(2006.01)i

According to International Patent Classification (IPC) or to both national classification and IPC

**B. FIELDS SEARCHED**

Minimum documentation searched (classification system followed by classification symbols)
Int.Cl. C09B, A23L, A61K8/00

Documentation searched other than minimum documentation to the extent that such documents are included in the fields searched
Published examined utility model applications of Japan 1922–1996
Published unexamined utility model applications of Japan 1971–2018
Registered utility model specifications of Japan 1996–2018
Published registered utility model applications of Japan 1994–2018

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2006-291075 A (SANEI GEN FFI INC.) 26 October 2006, paragraphs [0022]-[0057] (Family: none) | 1-12 |
| A | JP 2011-79792 A (JAPAN ALGAE CO., LTD.) 21 April 2011, paragraphs [0024]-[0034] & TW 201113025 A | 1-12 |
| A | WO 2013/105430 A1 (EZAKI GLICO CO., LTD.) 18 July 2013, claims & US 2014/0371433 A1: claims & CN 104039893 A & TW 201332456 A | 1-12 |

☒ Further documents are listed in the continuation of Box C.　　☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 November 2018 (08.11.2018) | 04 December 2018 (04.12.2018) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2018/034149

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2015-116335 A (ASAHI KASEI CHEMICALS CORP.) 25 June 2015, paragraphs [0043]-[0044] (Family: none) | 1-12 |
| A | JP 4-72364 A (KIKUKEN SENKO KK) 06 March 1992, examples 1-5 (Family: none) | 1-12 |

Form PCT/ISA/210 (continuation of second sheet) (January 2015)

**INTERNATIONAL SEARCH REPORT**

International application No.

PCT/JP2018/034149

The "naturally derived water-soluble pigment" in the invention in claim 1 includes all pigments obtained from natural substances regardless of the method used to obtain said pigments, but only the case in which a spirulina blue pigment is selected as the naturally derived water-soluble pigment is disclosed within the meaning of PCT Article 5. Thus, the invention in claim 1 lacks support within the meaning of PCT Article 6.

This also applies to the invention in claims 2-12.

Accordingly, the subject of the search in this search report was limited to the case in which a "spirulina blue pigment" is selected as the "naturally derived water-soluble pigment".

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004231851 A

- JP 2011099105 A

**Non-patent literature cited in the description**

- 17. Color Value Measurement Method. Japan's Specifications and Standards for Food Additives

- C. MONOGRAPHS. Japan's Specifications and Standards for Food Additives
- C. MONOGRAPHS. Japan's Specifications and Standards for Food Additives